# EUROPÄISCHE PATENTSCHRIFT

(11) **EP 0 838 450 B1**
(45) Veröffentlichungstag und Bekanntmachung des Hinweises auf die Patenterteilung: **20.02.2002**
(21) Anmeldenummer: 97118174.8
(22) Anmeldetag: 20.10.1997
(51) Int. Cl.: C07C 29/40, C07C 33/02

(54) **Verfahren zur Umsetzung von Grignardverbindungen mit Carbonylverbindungen und anschliessender Hydrolyse**
Method for the reaction of Grignard compounds with carbonyl compounds and subsequent hydrolysis
Procédé pour la réaction de composés de Grignard avec composés carbonyles et hydrolyse subséquent

(30) Priorität: 22.10.1996 DE 19643648
(43) Veröffentlichungstag der Anmeldung: 29.04.1998
(73) Patentinhaber: BASF AKTIENGESELLSCHAFT, 67056 Ludwigshafen (DE)
(72) Erfinder: Stroezel, Manfred, 68549 Ilvesheim (DE); Rheude, Udo, Dr., 67166 Otterstadt (DE); Rahn, Ralf-Thomas, Dr., 68167 Mannheim (DE); Kaibel, Gerd, Dr., 68623 Lampertheim (DE)
(74) Vertreter: Isenbruck, Günter, Dr.

(56) Entgegenhaltungen:
- EP-A- 0 073 569
- DE-A- 3 328 440
- US-A- 4 754 079
- DATABASE WPI Section Ch, Week 9007 Derwent Publications Ltd., London, GB; Class E17, AN 90-049554 XP002111777 & JP 02 004726 A (TAKASAGO PERFUMERY CO LTD), 9. Januar 1990 (1990-01-09)

## Beschreibung

Die vorliegende Erfindung betrifft die Durchführung von exothermen Reaktionen, ausgewählt aus der Anlagerungsreaktion von Grignardverbindungen an Carbonylverbindungen und der anschließenden Hydrolyse des entstehenden Additionsprodukts.

Nach dem Stand der Technik werden Grignardreaktionen mit Organomagnesiumhalogeniden in einem Lösungsmittel, z.B. Diethylether oder Tetrahydrofuran, durchgeführt, vgl. Übersichtsberichte in: Kirk-Othmer, Encyclopedia of Chemical Technology, 3rd Edition, John Wiley and Sons, 1980; und Houben-Weyl, Methoden der organischen Chemie, Metallorganische Chemie, Georg-Thieme-Verlag Stuttgart, 4. Auflage, 1973. Dazu wird Magnesium in unterschiedlicher Form (z.B. Barren, Brocken, Späne) mit in Lösungsmittel gelöstem Organohalogenid umgesetzt, wobei sich das Grignard-Reagenz bildet und in der Regel nicht umgesetzte Restmengen des Organohalogenids in der entstehenden Grignardlösung zurückbleiben. Dadurch geht wertvoller Einsatzstoff verloren. Hinzu kommt, daß die nachfolgende Additionsreaktion des Grignardreagenzes an die Carbonylverbindung zum Magnesiumalkoholat exotherm ist, so daß die freiwerdende Reaktionswärme durch Kühlung des Reaktionsgefäßes, in der Regel durch Kühlwasser, abgeführt werden muß. In einer darauf folgenden Hydrolysestufe wird das entstandene Magnesiumalkoholat hydrolysiert. Zur Abfuhr der bei der Hydrolyse entstehenden Wärme wird der Behälter ebenfalls gekühlt. Nachteilig bei der bisherigen Durchführung der Reaktion in Rührkesseln mit außenliegender Kühlung ist, daß wegen der notwendigen Wärmeabfuhr über die Wandfläche ein Apparat mit großer Wärmeübertragerfläche und daher großem Volumen erforderlich ist.

Aus DE-A-3 328 440 ist die Anwendung der Siedekühlung durch Verdampfen von im Reaktionsgemisch enthaltenem Lösungsmittel für die Herstellung von Iononen bekannt. Weiterhin ist die Siedekühlung bekannt zur Herstellung des Na-Salzes des 2-Nitropropandiols-1,3 aus DE-C-3 533 801, für die Flüssigphasenchlorierung von 1,3-Butadien aus EP-A-0 429 967 und für die Herstellung von Terephthalsäure aus EP-A-0 682 005. Nicht bekannt ist die Anwendung der Siedekühlung auf Grignardreaktionen.

Aus EP-A- 00 073 569 ist ein Verfahren zur Durchführung von Grignardreaktionen bekannt, wonach in einem ersten Verfahrensschritt die gesamte erforderliche Magnesiummenge und lediglich ein Anteil des erforderlichen Organohalogenids umgesetzt werden und anschließend in einem zweiten Reaktionsschritt das restliche Organohalogenid sowie die Carbonylverbindung zugesetzt werden, wobei die Grignardreaktion stattfindet und gleichzeitig weiteres Grignardreagenz in situ gebildet wird.

Die Aufgabe der vorliegenden Erfindung bestand nun darin, ein verbessertes Verfahren zur Umsetzung von Grignardverbindungen mit Carbonylverbindungen und der anschließenden Hydrolyse zu entwickeln, in apparativ weniger aufwendiger Weise.

Es wurde gefunden, daß sich dieses Problem lösen läßt, wenn man die bei der exothermen Anlagerungsreaktion der Grignardverbindungen an Carbonylverbindungen und der anschließenden exothermen Hydrolyse entstehende Wärme unter Ausnutzung der gleichzeitigen oder nachfolgenden Siedekühlung abführt, indem man ein Lösungsmittel oder Lösungsmittelgemisch mit bestimmten Siedeeigenschaften verwendet und dieses verdampft. Hierbei führt man die Anlagerungsreaktion bzw. Hydrolysereaktion bei Temperaturen durch, die etwa 0 bis 10 Grad oberhalb des Siedepunkts des Lösungsmittels bzw. Lösungsmittelgemischs liegen.

Somit betrifft die Erfindung ein Verfahren zur Durchführung einer oder mehrerer exothermer Reaktionen ausgewählt aus
(a) der Anlagerungsreaktion von Organomagnesiumhalogenidverbindungen der Formel RMgX, wobei R ein C₁-C₆-Alkyl-, C₂-C₆-Alkenyl- oder C₂-C₆-Alkinylrest oder ein Arylrest ist und X ein Cl, Br oder I ist, an eine Carbonylverbindung und
(b) der Hydrolyse von Additionsprodukten von Organomagnesiumhalogenidverbindungen der Formel RMgX, wobei R ein C₁-C₆-Alkyl-,C₂-C₆-Alkenyl- oder C₂-C₆-Alkinylrest oder ein Arylrest ist und X ein Cl, Br oder I ist, und einer Carbonylverbindung
in einem Lösungsmittel oder Lösungsmittelgemisch, das dadurch gekennzeichnet ist, daß man einen wesentlichen Teil der bei der Reaktion frei werdenden Wärme durch Verdampfung des Lösungsmittels oder Lösungsmittelgemisches abführt, das über Kopf abdestilliert wird, und das ein niedriger als das Lösungsmittel oder Lösungsmittelgemisch siedendes Organohalogenid der Formel RX, wobei R und X die obige Bedeutung haben, enthält.

Bevorzugte Ausführungsformen der Erfindung sind der nachfolgenden Beschreibung und der Figuren zu entnehmen. Weitere bevorzugte Ausführungsformen sind in den Unteransprüchen definiert.

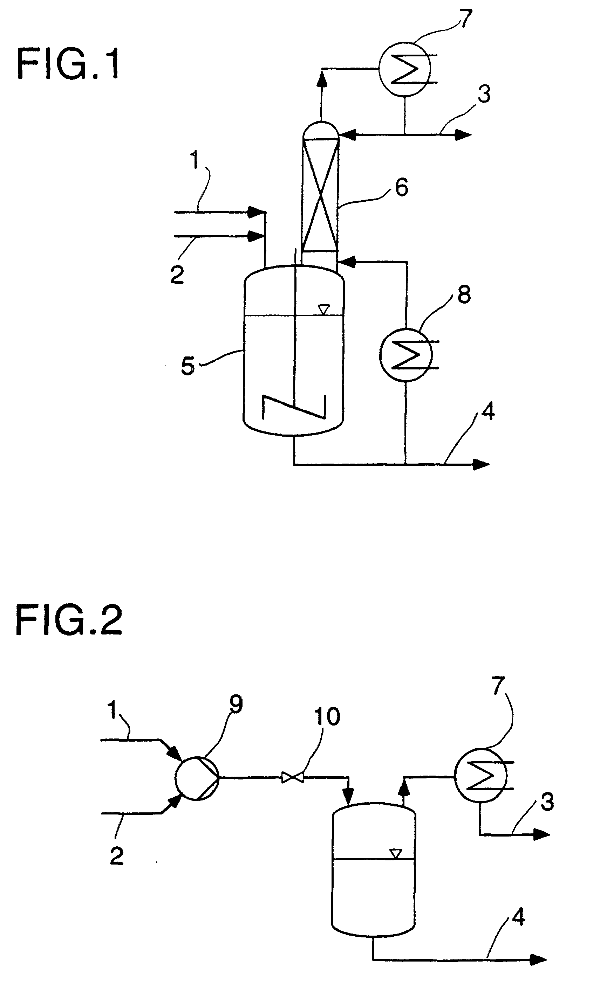

Die Figuren 1 bis 6 zeigen bevorzugte Ausführungsformen zur Durchführung des erfindungsgemäßen Verfahrens.

Bei den erfindungsgemäßen exothermen Reaktionen handelt es sich zum einen um die Anlagerung (Addition) von Organomagnesiumhalogenidverbindungen (Grignardverbindungen) der Formel RMgX, wobei R ein C₁-C₆-Alkyl-, C₂-C₆-Alkenyl-, oder C₂-C₆-Alkinylrest oder ein Arylrest ist, vorzugsweise ein Ethyl-, Vinyl- oder Ethinylrest, und X ein Cl, Br oder I ist, vorzugsweise Cl, an eine Carbonylverbindung, insbesondere eine C₁₋₄₀-Carbonylverbindung, stärker bevorzugt eine C₁₋₂₅-Verbindung, jeweils ausgewählt aus den entsprechenden Aldehyden, Ketonen und Estern ist, z.B. Geranylaceton. Bei der weiteren in Frage kommenden exothermen Reaktion handelt es sich um die Hydrolyse von Additionsprodukten aus Organomagnesiumhalogenidverbindungen der o.g. Formel RMgX, wobei die Gruppen R und X die obige Bedeutung haben, und einer Carbonylverbindung, die wie oben definiert ist. Im folgenden bezeichnet der Begriff Reaktion sowohl die Anlagerungsreaktion (Additionsreaktion) als auch die Hydrolysereaktion, soweit nichts anderes angegeben ist.

Erfindungsgemäß wird die Reaktion in einem Lösungsmittel oder Lösungsmittelgemisch durchgeführt, das unter den Reaktionsbedingungen zwischen 0 und 150°C, bevorzugt zwischen 25 und 80°C, insbesondere zwischen 50 und 70°C siedet und aus mindestens einem Ether und oder mindestens einem Amin, ggf. im Gemisch mit einem oder mehreren aliphatischen, cycloaliphatischen und/oder aromatischen Kohlenwasserstoffen, besteht. Bevorzugte Lösungsmittel sind Tetrahydrofuran (THF), Methyl-tert.-butyl-ether (MTBE), 1,4-Dioxan, Diethylether, Diphenylether und Anisol, wobei THF und MTBE am meisten bevorzugt sind. Bei der Hydrolysereaktion ist es meist vorteilhaft, dem Hydrolysewasser etwas Säure, vorzugsweise Schwefelsäure, zuzusetzen. Hierbei liegen insbesondere 20 bis 150 Gew.-%, bevorzugt 30 bis 80 Gew.-%, Wasser und 0,1 bis 5 Gew.-%, bevorzugt 0,5 bis 3 Gew.-%, Säure, vor, jeweils bezogen auf 100 Gew.-% Lösungsmittel bzw. Lösungsmittelgemisch.

Die bevorzugt eingesetzten Mengen an den verwendeten Komponenten betragen wie folgt: bei der Anlagerungsreaktion 1 bis 20 Gew.-%, insbesondere 3 bis 15 Gew.-%, Organomagnesiumhalogenidverbindung (Grignardreagenz) und 2 bis 40 Gew.-%, insbesondere 6 bis 30 Gew.-%, Carbonylverbindung; bei der Hydrolysereaktion 2 bis 40 Gew.-%, insbesondere 6 bis 30 Gew.-%, Additionsprodukt; Rest jeweils Lösungsmittel oder Lösungsmittelgemisch, wobei alle Angaben auf 100 Gew.-% Reaktoraustrag bei der Additions- bzw. Hydrolysereaktion bezogen sind. Im folgenden umfaßt der Begriff Lösungsmittel auch ein Lösungsmittelgemisch.

Das Lösungsmittel enthält ein niedriger als das Lösungsmittel siedendes Organohalogenid der Formel RX, wobei R und X die obige Bedeutung haben. Bei diesem Organohalogenid handelt es sich insbesondere um bei der Grignardreaktion nicht umgesetztes Restorganohalogenid. Das vorliegende Verfahren ermöglicht auf einfache und schonende Weise die Rückgewinnung dieses Organohalogenids.

In einer Ausführungsform der Erfindung wird die Reaktion bei einer Temperatur zwischen 0 und 150°C, vorzugsweise zwischen 25 und 80°C, insbesondere zwischen 50 und 70°C, durchgeführt. Bei dieser isothermen Fahrweise wird vorzugsweise bei einem Druck von 0,5 bis 10 bar, insbesondere 1 bis 3 bar, gearbeitet. In einer besonders bevorzugten Ausführungsform der Erfindung wird bei dieser Fahrweise während der Reaktion zusätzliches Lösungsmittel unter gleichzeitigem Beheizen zugeführt, was die weitere Rückgewinnung von o.g. Organohalogenid RX ermöglicht. Weiterhin ist es vorteilhaft, daß während der Reaktion leichtflüchtige Bestandteile der Reaktionsmischung, insbesondere Organohalogenid RX, mittels Dämpfen oder (Inert-)Gasen aus der Reaktionsmischung herausgestrippt werden.

Wird z.B. Vinylmagnesiumchlorid mit Ketonen in THF umgesetzt, erwies es sich als günstig, die Reaktion bei 50 bis 70°C, vorzugsweise bei 63 bis 68°C durchzuführen. Unter diesen Bedingungen gelang es, mehr als 70% des enthaltenen Vinylchlorids aus der Reaktionsmischung zurückzugewinnen. Das verdampfte Lösungsmittel wird zweckmäßigerweise wieder kondensiert. Um eine vollständige Abreicherung des Organohalogenids aus der Reaktionslösung zu erreichen, kann das Organohalogenid mit Inertgas oder Lösungsmitteldampf in einer Destillationskolonne aus dem Reaktionsaustrag herausgestrippt werden.

Die isotherme Fahrweise kann kontinuierlich, halbkontinuierlich oder diskontinuierlich durchgeführt werden, wobei sich ein Reaktor ohne, mit teilweiser oder gar vollständiger Rückvermischung, insbesondere ein kontinuierlich oder diskontinuierlich betriebener Rührkessel, Schlaufenreaktor oder Strahldüsenreaktor, eine kontinuierlich betriebene Kaskade aus Rührkesseln oder Schlaufenreaktoren oder Strahldüsenreaktoren eignet. Nach erfolgter Anlagerungsreaktion wird das Reaktionsprodukt vorteilhafterweise zuerst einer Destillationsstufe zugeführt, in der Organohalogenidreste aus dem Reaktoraustrag herausgestrippt werden, und dann der Hydrolysestufe zugeführt oder direkt in die Hydrolysestufe geführt.

In einer weiteren Ausgestaltung der Erfindung wird die exotherme Reaktion kontinuierlich unter adiabatischen Bedingungen durchgeführt. Da die adiabatische Temperaturerhöhung bei den exothermen Reaktionen beträchtlich sein kann und auch beträchtliche Drücke erreicht werden können, muß die Reaktion, um die Bildung von unerwünschten Nebenprodukten zu vermeiden, bei sehr kurzen Verweilzeiten erfolgen. So kann z.B. im Falle der Anlagerung von Vinylmagnesiumchlorid an Ketone in THF je nach Reaktionsbedingungen und Konzentration des Lösungsmittels die Temperaturerhöhung 30 bis 100 K betragen, so daß die Temperaturen mehr als 100°C erreichen können und die Drücke höher als 10 bar liegen können. Als Apparate eignen sich alle Reaktoren, die die Durchführung der Reaktion bei kurzen Verweilzeiten erlauben. Zweckmäßigerweise werden z.B. Reaktionspumpen, wie z.B. in der deutschen Patentanmeldung P 42 20 239 beschrieben, Mischdüsen, statische Mischer, Dispergiermaschinen oder dynamische Durchflußmischer mit einer Verweilzeitstrecke von 1 - 100 s, eingesetzt.

Bei der adiabatischen Fahrweise wird die Reaktion bei einer Temperatur von 0 bis 200°C, vorzugsweise von 20 bis 100°C, insbesondere von 30 bis 80°C, einem Druck von 1 bis 20 bar, vorzugsweise von 2 bis 10 bar, insbesondere von 3 bis 7 bar, und einer Verweilzeit von 0,1 bis 100 s, vorzugsweise 2 bis 10 s, insbesondere von 3 bis 8 s, durchgeführt. Nach erfolgter Reaktion wird das Produkt auf einen niedrigeren Druck entspannt, vorzugsweise in einer "*Entspannungszone*" (Flash-Zone) mittels eines Entspannungsventils in ein Entspannungsgefäß hinein. Zweckmäßigerweise wird der bei der Entspannung anfallende Brüden dann kondensiert. Da bei der adiabatischen Fahrweise sehr hohe Temperaturen erreicht werden können, ist es vorteilhaft, die umzusetzenden Ausgangsstoffe vor der Reaktion abzukühlen. In einer bevorzugten Ausführungsform werden leichtflüchtige Bestandteile, insbesondere Organohalogenid RX, nach erfolgter Reaktion aus dem Reaktoraustrag mittels Dämpfen oder (Inert-)Gasen herausgestrippt oder analog zur isothermen Fahrweise zusätzliches Lösungsmittel unter gleichzeitigem Beheizen zugeführt. Somit kann auch hier nicht umgesetztes Organohalogenid durch Strippen der Reaktionslösung mit gas- oder dampfförmigen Strippmedien in einer Destillationskolonne abgetrennt werden.

Vorzugsweise wird die Strippung von leichtflüchtigen Bestandteilen in einer Destillationskolonne durchgeführt. Allgemein eignen sich als Destillationskolonnen hierfür und zur Aufreinigung des Reaktionsprodukts der Anlagerungs- oder Hydrolysereaktion Kolonnen mit feststoffunempfindlichen Einbauten, z.B. Dual-Flow-Böden, Glocken- oder Ventilböden oder spezielle feststoffunempfindliche, geordnete Blech-, Glas- oder Porzellan-Packungen oder Füllkörper.

Das erfindungsgemäße Verfahren ermöglicht die Durchführung der Reaktionen in Apparaten mit geringem Volumen und gleichzeitig eine Rückgewinnung von niedriger als das Lösungsmittel oder das Lösungsmittelgemisch siedendem Organohalogenid RX. Diese Rückgewinnung des leicht flüchtigen Organohalogenids kann, soweit sie nicht oder nur unvollständig bei der Anlagerungsreaktion stattgefunden hat, in der Hydrolysestufe erfolgen. Allerdings ist dies weniger bevorzugt, da die Destillatfraktion mit dem Organohalogenid neben dem Lösungsmittel auch noch Wasser enthält, das mittels eines weiteren Verfahrensschritts entfernt werden muß, bevor die Destillatfraktion vorteilhafterweise in die Herstellungsstufe der Grignardverbindung zurückgeführt wird.

Ein weiterer Vorteil des erfindungsgemäßen Verfahrens gegenüber Verfahren mit einer herkömmlichen Kühlung des Rührkessels von außen liegt auch darin, daß Feststoffablagerungen am Wärmetauscher vermieden werden können. Damit sind die bisher verwendeten Systeme mit außen liegender Kühlung nicht mehr erforderlich.
- Fig. 1: zeigt eine für die isotherme Reaktionsführung geeignete Reaktionsapparatur aus einem Rührkessel (5) mit aufgesetzter Kolonne (6), Kondensator (7) und Verdampfer (8). Über die Leitungen (1) und (2) werden die Ausgangsstoffe, d.h. entweder die Organomagnesiumhalogenidverbindung (Grignardreagenz), eine Carbonylverbindung und Lösungsmittel/-gemisch oder die Anlagerungsverbindung, Wasser und Lösungsmittel/-gemisch, ggf. mit Säure, in den Rührkessel (5) zugeführt und dort umgesetzt. Über Leitung (4) wird das Reaktionsprodukt abgezogen, während das verdampfte Lösungsmittel/ -gemisch im Gemisch mit Organohalogenid, destilliert und dann über Leitung (3) abgezogen wird. Ein geeigneter Reaktor (5) ist ein Rührkessel oder ein Schlaufenreaktor, ggf. mit Mischdüse.
- Fig. 2: zeigt eine Reaktionspumpe (9), in die die Ausgangsstoffe über Leitungen (1) und (2) zugeführt und dort umgesetzt werden. Das Reaktionsprodukt wird dann über das Entspannungsventil (10) entspannt und in einen Behälter geleitet, in dem eine Trennung in flüssige und dampfförmige Stoffe erfolgt. In Fig. 2 bedeuten gleiche Bezugsziffern wie in Fig. 1 das gleiche. Bei der in Fig. 2 dargestellten adiabatischen Fahrweise kann nicht umgesetztes Organohalogenid durch Strippen mit gas- oder dampfförmigen Strippmedien im Behälter oder in einer dem Entspannungsventil nachgeschalteten Destillationskolonne (nicht gezeigt) abgetrennt werden.

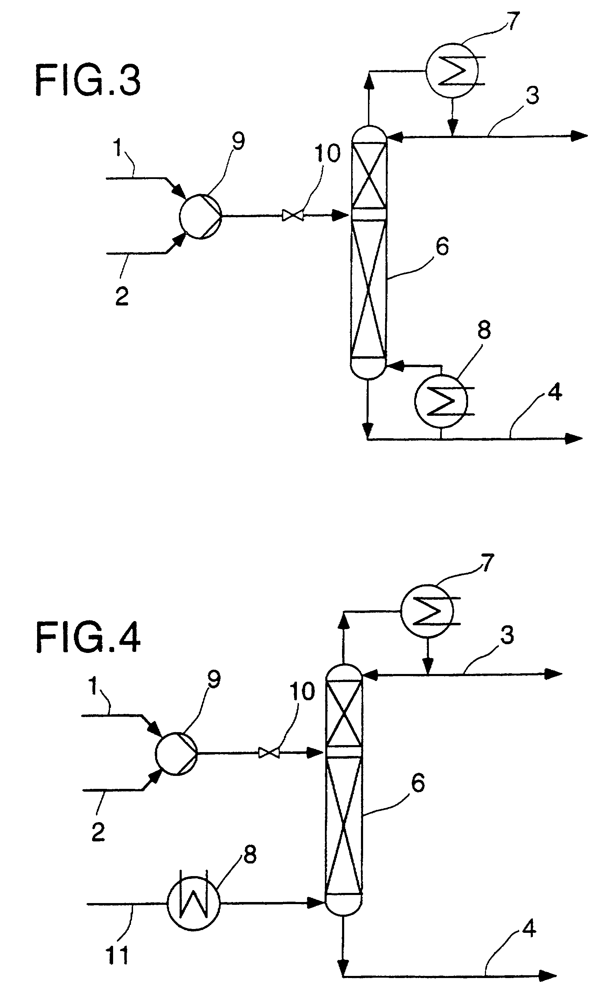

Bei den nachfolgenden Figuren 3 bis 6 bedeuten gleiche Bezugsziffern wie in den Figuren 1 und 2 das gleiche.

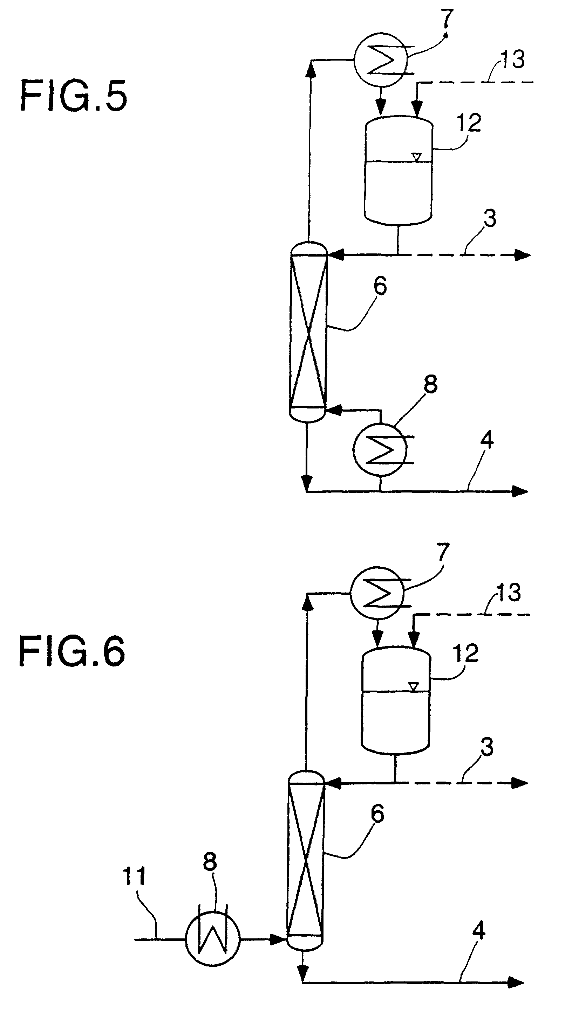

Fig. 3 zeigt wiederum die adiabatische Fahrweise, wobei der Reaktoraustrag nach dem Entspannen in die Destillationskolonne (6) eingespeist wird, um aus dem Sumpf der Kolonne im wesentlichen organohalogenidfreies Produkt über Leitung (4) abziehen zu können. Auf diese Weise können im Sumpfprodukt Organohalogenidgehalte im ppm-Bereich erreicht werden. Um eine zu starke Einengung des Sumpfprodukts, die zu einer Ausfällung des Magnesiumalkoholats führen kann, zu verhindern, ist die Zufuhr von zusätzlichem Lösungsmittel (11) am Kolonnensumpf, wie in Fig. 4 gezeigt, zweckmäßig. Neben einer Aufwärtsfahrweise, bei der das Reaktionsprodukt in eine Destillationsblase geleitet wird, aus der heraus die Leichtsieder abdestilliert werden, erweist sich die in Fig. 5 gezeigte Abwärtsfahrweise als vorteilhaft, bei der der Reaktoraustrag (13) über den Vorlagebehälter (12) auf den Kopf einer diskontinuierlich betriebenen Destillationskolonne (6) aufgegeben wird. Hierbei wird das organohalogenidreiche Destillat (3) am Kopf abgezogen und wieder in den Vorlagebehälter (12) gegeben, in dem eine Vermischung mit weiterem Reaktoraustrag (13) erfolgt. Das praktisch organohalogenidfreie Sumpfprodukt (4) wird abgezogen. Auch hier kann eine Zufuhr von frischem Lösungsmittel (11) am Kolonnensumpf unter gleichzeitiger Beheizung, wie in Fig. 6 gezeigt, vorteilhaft sein, um weiteres Organohalogenid rückgewinnen zu können.

Die Erfindung wird anhand der folgenden Beispiele, die bevorzugte Ausführungsformen der Erfindung darstellen, näher erläutert.

### BEISPIELE

### Beispiel 1 (Anlagerungsreaktion)

Innerhalb von 1 h werden 3500 g einer 50°C warmen Lösung, die 4,6 Gew.-% Vinylchlorid, 89,0 Gew.-% THF und 6,4 Gew.-% Vinylmagnesiumchlorid enthält, mit 497 g Geranylaceton (Molmasse: 194) (Eingangstemperatur 50°C) kontinuierlich in einem Rührkessel bei 65°C umgesetzt. Dabei fällt ein Destillat von 815 g mit einer Zusammensetzung von 15 Gew.-% Vinylchlorid und 85 Gew.-% THF sowie ein flüssiger Reaktoraustrag von 3182 g mit einer Zusammensetzung von 76,1 Gew.-% THF, 1,2 Gew.-% Vinylchlorid und 22,6 Gew.-% des Additionsprodukts Chloromagnesium-Nerolidolat (Molmasse: 281) an. Im Destillat werden 76% des im Feedstrom enthaltenen Vinylchlorids zurückgewonnen. Eine externe Kühlung des Rührkessels mit Kühlwasser ist nicht erforderlich.

### Beispiel 2 (Anlagerungsreaktion)

Wie in Beispiel 1 werden innerhalb von 1 h 3500 g einer 50°C warmen Lösung, die 4,6 Gew.-% Vinylchlorid, 89,0 Gew.-% THF und 6,4 Gew.-% Vinylmagnesiumchlorid enthält, mit 497 g Geranylaceton (Molmasse: 194) (Eingangstemperatur 50°C) kontinuierlich in einem Rührkessel bei 65°C umgesetzt. Zusätzlich werden in den Reaktionskessel 1000 g flüssiges THF gepumpt und der Kessel mit einer Heizleistung von etwa 140 W so beheizt, daß die gleiche Sumpfaustragsmenge erhalten wird, wie wenn kein zusätzliches THF zugeführt worden wäre. Es fällt ein Destillat von 1815 g mit einer Zusammensetzung von 7,7 Gew.-% Vinylchlorid und 92,3 Gew.-% THF sowie ein flüssiger Reaktoraustrag von 3182 g mit einer Zusammensetzung von 76,1 Gew.-% THF, 1,2 Gew.-% Vinylchlorid und 22,6 Gew.-% des Additionsprodukts Chloromagnesium-Nerolidolat (Molmasse: 281) an. Im Destillat werden 87% des im Feedstrom enthaltenen Vinylchlorids zurückgewonnen.

### Beispiel 3 (Strippung des Reaktoraustrags einer Anlagerungsreaktion)

Auf den Kopf einer Kolonne mit einer strukturierten Packung von 1,10 m Länge und einem Durchmesser von 50 mm werden 757 g/h einer Mischung aus 3,5 Gew.-% Vinylchlorid, 66 Gew.-% THF, 28,2 Gew.-% des Additionsprodukts Chloromagnesium-Nerolidolat (Molmasse: 281) sowie 1,7 Gew.-% weitere Hochsieder gegeben. Dem Kolonnensumpf werden 594 g/h reines THF in dampfförmiger Form zugeführt. Bei einem Rücklaufverhältnis von 2 wird ein Destillat von 552 g/h mit 95,2 Gew.-% THF und 4,8 Gew.-% Vinylchlorid sowie ein flüssiges Sumpfprodukt von 799 g/h mit einer Zusammensetzung von 71,1 Gew.-% THF, 27,3 Gew.-% Additionsprodukt, 100 ppm Vinylchlorid sowie 1,6 Gew.-% Hochsieder gewonnen. Im Destillat werden > 99% des im Feed enthaltenen Vinylchlorids zurückgewonnen.

### Beispiel 4 (Hydrolysereaktion)

Innerhalb von 1 h werden in einem Rührkessel 3792 g einer 60°C warmen Lösung, die 79,1 Gew.-% THF, 2,8 Gew.-% Vinylmagnesiumchlorid, 17,4 Gew.-% Additionsprodukt Chloromagnesium-Nerolidolat (Molmasse: 281) sowie 0,9 Gew.-% Vinylchlorid enthält, mit 1814 g ca. 2 Gew.-%iger Schwefelsäure, die eine Temperatur von 36°C hat, versetzt, und das Additionsprodukt wird hydrolysiert. Bei der Hydrolyse fällt ein Destillat von ca. 130 g/h mit einer Temperatur von 55°C an, das 64 Gew.-% THF, 5 Gew.-% Wasser, 13 Gew.-% Ethylen und 18 Gew.-% Vinylchlorid enthält. Eine externe Kühlung des Rührkessels mit Kühlwasser ist nicht erforderlich. Im Destillat werden ca. 70 % des eingesetzten Vinylchlorids abgetrennt und können einer Aufarbeitungsstufe zugeführt werden.

## Patentansprüche

1. Verfahren zur Durchführung von einer oder mehreren exothermen Reaktionen ausgewählt aus
(a) der Anlagerungsreaktion von Organomagnesiumhalogenidverbindungen der Formel RMgX, wobei R ein C₁-C₆-Alkyl-, C₂-C₆-Alkenyl- oder C₂-C₆-Alkinylrest oder ein Arylrest ist und X Cl, Br oder I ist, an eine Carbonylverbindung und
(b) der Hydrolyse von Additionsprodukten von Organomagnesiumhalogenidverbindungen der Formel RMgX, wobei R ein C₁-C₆-Alkyl-, C₂-C₆-Alkenyl- oder C₂-C₆-Alkinylrest oder ein Arylrest ist und X Cl, Br oder I ist, an eine Carbonylverbindung
in einem Lösungsmittel oder Lösungsmittelgemisch, **dadurch gekennzeichnet, daß** man einen wesentlichen Teil der bei der Reaktion frei werdenden Wärme durch Verdampfung des Lösungsmittels oder Lösungsmittelgemisches abführt, das über Kopf abdestilliert wird, und das ein niedriger als das Lösungsmittel oder Lösungsmittelgemisch siedendes Organohalogenid der Formel RX, wobei R und X die obige Bedeutung haben, enthält.

2. Verfahren nach Anspruch 1, **dadurch gekennzeichnet, daß** das Lösungsmittel oder Lösungsmittelgemisch unter den Reaktionsbedingungen einen Siedepunkt zwischen 0 und 150°C, insbesondere zwischen 25 und 80°C, hat und mindestens einen Ether und/oder mindestens ein Amin, gegebenenfalls. im Gemisch mit einem oder mehreren aliphatischen, cycloaliphatischen und/oder aromatischen Kohlenwasserstoffen, enthält.

3. Verfahren nach Anspruch 1 oder 2, **dadurch gekennzeichnet, daß** die Reaktion bei einer Temperatur zwischen 0 und 150°C, insbesondere zwischen 25 und 80°C, durchgeführt wird.

4. Verfahren nach Anspruch 3, **dadurch gekennzeichnet, daß** die Reaktion bei einem Druck von 0,5 bis 10 bar, insbesondere 1 bis 3 bar, durchgeführt wird.

5. Verfahren nach Anspruch 3 oder 4, **dadurch gekennzeichnet, daß** während der Reaktion leichtflüchtige Bestandteile der Reaktionsmischung, insbesondere Organohalogenid RX, mittels Dämpfen oder Gasen aus der Reaktionsmischung herausgestrippt werden.

6. Verfahren nach Anspruch 1 oder 2, **dadurch gekennzeichnet, daß** die Reaktion kontinuierlich bei einer Temperatur von 0 bis 200 °C, insbesondere von 20 bis 100°C, einem Druck von 1 bis 20, insbesondere von 2 bis 10 bar, und einer Verweilzeit von 0,1 bis 100 s, vorzugsweise von 2 bis 10 s, durchgeführt wird und nach erfolgter Reaktion das Produkt auf einen niedrigeren Druck entspannt wird.

7. Verfahren nach Anspruch 6, **dadurch gekennzeichnet, daß** bei der Entspannung anfallende Brüden kondensiert wird.

8. Verfahren nach Anspruch 6 oder 7, **dadurch gekennzeichnet, daß** leichtflüchtige Bestandteile, insbesondere Organohalogenid RX, nach erfolgter Reaktion aus dem Reaktoraustrag mittels Dämpfen oder Gasen herausgestrippt werden.

9. Verfahren nach Anspruch 5 oder 8, **dadurch gekennzeichnet, daß** die Strippung in einer Destillationskolonne durchgeführt wird.

## Claims

1. A process for carrying out one or more exothermal reactions selected from
(a) the addition reaction of organomagnesium halide compounds of the formula RMgX, where R is C₁-C₆-alkyl, C₂-C₆-alkenyl or C₂-C₆-alkynyl or aryl and X is Cl, Br or I, to a carbonyl compound and
(b) the hydrolysis of addition products of organomagnesium halide compounds of the formula RMgX, where R is C₁-C₆-alkyl, C₂-C₆-alkenyl or C₂-C₆-alkynyl or aryl and X is Cl, Br or I, to a carbonyl compound
in a solvent or solvent mixture, wherein a considerable fraction of the heat liberated during the reaction is removed by evaporation of the solvent or solvent mixture which is distilled off at the head and which contains an organohalide whose boiling point is lower than that of the solvent or solvent mixture and which is of the formula RX, where R and X have the abovementioned meanings.

2. The process as claimed in claim 1, wherein the solvent or solvent mixture under the reaction conditions has a boiling point of between 0 and 150°C, in particular between 25 and 80°C, and contains at least one ether and/or at least one amine, which may be mixed with one or more aliphatic, cycloaliphatic and/or aromatic hydrocarbons.

3. The process as claimed in claim 1 or 2, wherein the reaction is carried out at between 0 and 150°C, in particular between 25 and 80°C.

4. The process as claimed in claim 3, wherein the reaction is carried out at from 0.5 to 10 bar, in particular from 1 to 3 bar.

5. The process as claimed in claim 3 or 4, wherein highly volatile components of the reaction mixture, in particular organohalides RX, are stripped from the reaction mixture, during the reaction, by means of vapors or gases.

6. The process as claimed in claim 1 or 2, wherein the reaction is carried out continuously at from 0 to 200°C, in particular from 20 to 100°C, at from 1 to 20, in particular from 2 to 10 bar and a residence time of from 0.1 to 100 s, preferably from 2 to 10 s, and after the reaction the product is depressurized.

7. The process as claimed in claim 6, wherein vapor produced in the course of flashing is condensed.

8. The process as claimed in claim 6 or 7, wherein highly volatile components of the reaction mixture, in particular organohalide RX, are stripped from the reactor output, after the reaction, by means of vapors or gases.

9. The process as claimed in claim 5 or 8, **characterized in that** said stripping is carried out in a distillation column.

## Revendications

1. Procédé pour effectuer une ou plusieurs réactions exothermiques choisies parmi
a) la réaction d'addition de composés organomagnésiens halogénés de formule RMgX, où R représente un radical alkyle en C₁ à C₆, alcényle en C₂ à C₆ ou alkinyle en C₂ à C₆, ou un radical aryle, et X représente Cl, Br ou I, sur un composé carbonyle, et
b) l'hydrolyse de produits d'addition de composés organomagnésiens halogénés de formule RMgX, où R représente un radical alkyle en C₁ à C₆, alcényle en C₂ à C₆ ou alkinyle en C₂ à C₆, ou un radical aryle, et X représente Cl, Br ou I, sur un composé carbonyle
dans un solvant ou un mélange de solvants, **caractérisé en ce que** l'on évacue une partie de la chaleur libérée par la réaction par évaporation du solvant ou du mélange de solvants, laquelle partie est évacuée par distillation en tête de colonne, et contient un halogénure organique de formule RX à point d'ébullition inférieur à celui du solvant ou du mélange de solvants, et où R et X ont les significations ci-dessus.

2. Procédé selon la revendication 1, **caractérisé en ce que** le solvant ou le mélange de solvants présentent, dans les conditions de la réaction, un point d'ébullition compris entre 0 et 150°C, en particulier entre 25 et 80°C, et contiennent au moins un éther et/ou au moins une amine, éventuellement en mélange. avec un ou plusieurs hydrocarbures aliphatiques, cycloaliphatiques et/ou aromatiques.

3. Procédé selon la revendication 1 ou 2, **caractérisé en ce que** la réaction est entreprise à des températures de 0 à 150°C, en particulier de 25 à 80°C.

4. Procédé selon la revendication 3, **caractérisé en ce que** la réaction est entreprise à une pression de 0,5 à 10 bar, en particulier de 1 à 3 bar.

5. Procédé selon la revendication 3 ou 4, **caractérisé en ce que**, pendant la réaction, les composants volatils du mélange réactionnel, en particulier l'halogénure organique RX, sont extraits du mélange réactionnel par strippage, au moyen de vapeurs ou de gaz.

6. Procédé selon la revendication 1 ou 2, **caractérisé en ce que** la réaction est entreprise en continu, à des températures de 0 à 200°C, en particulier de 20 à 100°C, une pression de 1 à 20, en particulier de 2 à 10 bar, et avec un temps de séjour de 0,1 à 100 s, de préférence de 2 à 10 s, et qu'au terme de la réaction le produit est détendu à faible pression.

7. Procédé selon la revendication 6, **caractérisé en ce que** les fumées formées lors de la détente sont condensées.

8. Procédé selon la revendication 6 ou 7, **caractérisé en ce que** les composants volatils, en particulier l'halogénure organique RX, sont extraits du mélange réactionnel par strippage, au moyen de vapeurs ou de gaz.

9. Procédé selon la revendication 5 ou 8, **caractérisé en ce que** le strippage est entrepris dans une colonne de distillation.
